# DEMANDE DE BREVET EUROPEEN

(11) **EP 1 043 337 A1**
(43) Date de publication de la demande: **11.10.2000**
(21) Numéro de dépôt: 00400935.3
(22) Date de dépôt: 05.04.2000
(51) Int. Cl.: C08B 30/10, C12N 1/14, C12N 1/16, C12N 1/20

(54) **Corn-steep pulverulent**

(30) Priorité: 08.04.1999 FR 9904392
(71) Demandeur: Roquette Frères, 62136 Lestrem (FR)
(72) Inventeur: Erpicum, Thomas, Seraing, Liège 4100 (BE); Saniez, Marie-Hélène, 59350 Saint Andre (FR)
(74) Mandataire: Boulinguiez, Didier

(57) **Abrégé**

L'invention concerne un corn-steep pulvérulent sans support de séchage caractérisé en ce qu'il contient de l'acide lactique.

Elle concerne également un procédé d'obtention de ce corn-steep pulvérulent ainsi que son utilisation dans l'industrie de la fermentation.

## Description

La présente invention concerne un corn-steep pulvérulent sans support de séchage.

Elle a trait également à un procédé particulier d'obtention de ce corn-steep pulvérulent et aux utilisations de celui-ci dans l'industrie.

Le corn-steep, terme fréquemment utilisé par l'homme de l'art, désigne les eaux de trempage concentrées résultant de la trempe du maïs.

La trempe du maïs dans l'eau constitue la première étape de l'extraction de l'amidon en amidonnerie humide. Cette trempe permet le gonflement des grains de maïs et l'élimination de matières solubles hautement fermentescibles contenues dans ces grains. Elle consiste à maintenir le maïs placé dans des silos pendant un temps donné au sein d'une eau chaude contenant une faible quantité d'anhydride sulfureux, ceci afin de faciliter la séparation ultérieure protéïnes-cellulose-amidon, et d'empêcher par ailleurs la croissance de microorganismes indésirables.

Deux phénomènes essentiels se déroulent simultanément au cours de la trempe : le premier consiste en une diffusion des matières solubles du grain de maïs vers l'eau de trempe, alors que la deuxième consiste en une fermentation de ces matières solubles dans l'eau de trempe par des bactéries lactiques, les conditions de trempe (présence de sulfites, de sucres réducteurs, température) étant favorables au développement rapide de cette flore bactérienne.

Le principal intérêt de ces eaux de trempe concentrées, appelées couramment corn-steep par l'homme du métier, tient à leur composition en matières nutritives essentielles, issues du transfert des matières solubles du grain. Ces matières nutritives constituent des facteurs favorables à la croissance des microorganismes comme à la production de métabolites secondaires, et font du corn-steep une source idéale de substances nutritives dans les industries de fermentation.

En effet, le corn-steep constitue une source d'azote organique de choix par la répartition et les formes de ses acides aminés : libres, peptidiques, protéiques ainsi qu'une source de carbone (acide lactique) et de phosphate (acide phytique) à effet retard. Une teneur importante en vitamines et oligo-éléments complète l'intérêt que représente le corn-steep comme source nutritive pour la croissance de microorganismes et l'induction de métabolites secondaires lorsque le corn-steep est associé à une ou plusieurs sources de carbone (glucose, maltodextrine, amidon, saccharose .... ) .

En outre, il constitue une source nutritive relativement peu coûteuse, comparativement aux extraits de levures qui représentent la matière de référence dans ce domaine, et qui sont utilisés également en alimentation humaine et animale.

Par ailleurs, il est connu que l'utilisation du corn-steep en remplacement des sources d'azote complexes telles que les protéïnes de coton ou de soja, permet d'augmenter de manière substantielle les rendements de production d'antibiotiques.

Cependant, le corn-steep sous forme liquide pose des problèmes de décantation au cours du temps, ce qui est particulièrement gênant pour le transport, le stockage et le pompage du produit. Celui-ci doit être stocké en cuves agitées, thermostatées, pour limiter l'évolution de sa composition. Ceci est d'autant plus vrai lorsque le corn-steep est consommé par petites quantités à la fois par certaines industries de fermentation. Il est dans ce cas particulièrement important de maîtriser sa conservation. L'obtention de formes sèches de corn-steep a alors été envisagée.

Le corn-steep contient 45 à 55% d'eau. Une évaporation pour éliminer plus de la moitié de l'eau qu'il contient n'est pas envisageable en raison du coût de l'évaporation d'une part, et de la sensibilité de certains nutriments à la chaleur d'autre part.

Le séchage par atomisation du corn-steep liquide a alors été étudié.

Cette technique, très largement répandue dans l'industrie, a permis le séchage de produits réputés difficiles à sécher, tels que les produits thermosensibles ou fortement hygroscopiques. Cependant, dans le cas du corn-steep, l'homme de l'art s'est heurté à des problèmes de collage. En effet, si le séchage de certains produits ne pose aucun problème, c'est à dire que la poudre sort régulièrement de la chambre ou tour de séchage et présente un aspect et des propriétés d'écoulement satisfaisants, en revanche le corn-steep liquide, riche en acides aminés et en substances hygroscopiques, est sujet au collage.

Ce collage se manifeste par des dépôts à différents niveaux de la chambre de séchage et des matériels annexes, dépôts qui génèrent des pertes et une dégradation du produit, qui peuvent nécessiter des arrêts de fabrication avec des cycles de nettoyage coûteux, en temps, en matière et en main d'oeuvre. Le collage peut aussi survenir en aval du séchage lorsque la poudre après une durée de stockage plus ou moins longue perd sa fluidité et forme des mottes.

Il est donc très difficile d'atomiser du corn-steep, en raison de la nature particulièrement hygroscopique des acides organiques et des sels qu'il contient, et en particulier du lactate de potassium présent en forte proportion (Corn Chemistry and Technology - K.N. WRIGHT 466-467).

Pour pallier ces problèmes de collage, des méthodes parfois empiriques ont été appliquées au stade industriel, telles que l'ajout de supports de séchage au produit à atomiser, d'agents anti-agglomérants ou de dessicants en poudre.

Dans les industries agro-alimentaires, des supports fréquemment employés sont les maltodextrines et les sirops de glucose. Toutefois, leurs taux d'incorporation sont limités par l'effet diluant qu'ils exercent sur le produit et par leur coût élevé. De plus, de tels produits ne peuvent subir un traitement thermique ultérieur, comme une stérilisation, car leur teneur élevée en sucres libres fait qu'ils subissent une caramélisation. En outre ces compositions sont fortement hygroscopiques, ce qui entraîne des phénomènes de mottage lors de leur stockage.

Il a alors été proposé, dans le brevet US 2.859.114, de mélanger des fibres de maïs au corn-steep, de façon à ce qu'il soit absorbé par ces fibres, rendant alors possible une opération de séchage. Ce procédé a cependant le désavantage d'apporter au corn-steep une forte teneur en matières insolubles, ce qui n'est pas souhaitable dans les applications envisagées, notamment en fermentation.

En effet, un tel produit présente très vite des phénomènes de décantation lors du stockage et par là même, une répartition hétérogène des substances nutritives.

De plus, le produit présente en final une concentration de corn-steep insuffisante.

D'autres solutions, comme celle décrite dans le brevet US 3.655.396 consistent à éliminer complètement l'acide lactique du corn-steep. L'acide lactique ayant en effet été mis en cause dans les problèmes de séchage du corn-steep sans que l'on ne l'explicite véritablement, celui-ci est éliminé au moyen de levures capables de le métaboliser, le milieu de culture obtenu étant ensuite séché sur tambours ou par atomisation.

Cependant, un tel procédé a pour inconvénient de priver le corn-steep d'une source importante de carbone d'une part, mais aussi d'un précurseur particulièrement intéressant dans la culture de micro-organismes diauxiques.

Il existait donc un besoin en une forme sèche de corn-steep, de valeur nutritive et de solubilité préservées.

Forte de ce constat, la Demanderesse a donc cherché à mettre au point un corn-steep pulvérulent dont les qualités soient préservées, et n'ayant pas les défauts précédemment décrits des corn-steep en poudre selon l'art antérieur.

Et il est du mérite de la Demanderesse d'avoir réussi, contre toute attente, après avoir mené une recherche approfondie sur le sujet, à préparer un corn-steep pulvérulent sans être confrontée à des problèmes de collage, ce corn-steep pulvérulent ne présentant pas les défauts relevés pour les corn-steep en poudre de l'art antérieur, tout en ayant conservé ses qualités initiales. Elle a mis en évidence, de manière surprenante et inattendue, qu'un tel corn-steep pulvérulent pouvait être préparé sans support de séchage et sans traitement de séparation préalable, dans des conditions particulières à partir de corn-steep liquide, par un procédé d'atomisation, lequel n'avait jamais permis dans le passé d'obtenir directement et sans collage des corn-steep en poudre de qualité nutritionnelle intacte voire améliorée, de solubilité satisfaisante, et n'ayant pas tendance à motter.

Cela est d'autant plus surprenant que l'atomisation du corn-steep liquide apparaissait, selon les brevets US 2 859 114 et 3 655 396, impossible sans support de séchage ou sans éliminer une fraction du corn-steep.

L'invention se rapporte donc en premier lieu à un corn-steep pulvérulent sans support de séchage. on désigne par support de séchage les composés organiques solubles ou insolubles, susceptibles d'absorber le corn-steep liquide ou d'en modifier l'activité en eau. Ces supports connus de l'homme du métier sont notamment les fibres végétales, les drêches, les sons, les protéines, les polysaccharides, les carbohydrates, les dessicants.

La caractéristique essentielle du corn-steep pulvérulent selon l'invention tient au fait qu'il contient de l'acide lactique. On entend par acide lactique les formes D et L de l'acide lactique, ainsi que ses sels. De préférence, cette teneur en acide lactique est supérieure ou égale à 5% en poids sur sec, et encore plus préférentiellement supérieure ou égale à 10% en poids sur sec. Il conserve par conséquent ses qualités par rapport aux corn-steep pulvérulents de l'art antérieur dont on aurait éliminé l'acide lactique, et ne présente pas les inconvénients d'insolubilité ou de caramélisation des corn-steep de l'art antérieur, séchés sur supports de type fibres cellulosiques ou maltodextrines.

De manière avantageuse, le corn-steep pulvérulent selon l'invention comprend une teneur en ions métalliques comprise entre 1 et 5% en poids (sec/sec). De préférence, cette teneur est comprise entre 2 et 4% en poids (sec/sec).

La Demanderesse a en effet mis en évidence, après de longues recherches, que cette seconde caractéristique permettait avantageusement d'obtenir un corn-steep pulvérulent ayant conservé ses propriétés et ses qualités nutritives initiales.

Ainsi, au delà de 5% en poids d'ions métalliques, le corn-steep devient inutilisable dans un milieu de fermentation puisqu'il apporte un excès de métaux défavorable à la croissance des microorganismes. D'autre part, en deçà de 1% d'ions métalliques, il n'est pas possible d'obtenir un corn-steep pulvérulent sans l'aide d'un support de séchage ou sans éliminer certains de ses éléments constitutifs, comme l'acide lactique.

On préfère que les ions métalliques soient choisis dans le groupe constitué par les sels de zinc, de magnésium, de calcium, de strontium, de baryum ou de lithium, seuls ou en mélange entre eux. En particulier, les sels de magnésium conviennent parfaitement. En effet, ceux-ci constituent un apport en ions Mg2+ dont on connaît le rôle fondamental dans la croissance et le métabolisme de nombreux microorganismes. Ce cation est en effet vital dans la stabilisation de la structure cellulaire, et impliqué dans la plupart des mécanismes métaboliques en tant que cofacteur enzymatique.

De ce fait, les qualités nutritives du corn-steep pulvérulent selon l'invention s'en trouvent non seulement préservées, mais aussi améliorées. On comprend alors que ces qualités nutritives, alliées à la facilité de stockage et de mise en oeuvre soient un avantage indéniable, par exemple dans la préparation de milieux de culture destinés à la fermentation.

Le corn-steep pulvérulent conforme à l'invention possède également d'autres caractéristiques avantageuses. On peut citer sa très bonne aptitude à être comprimé, et sa très bonne aptitude à être mélangé à d'autres produits.

L'invention a trait en second lieu à un procédé de préparation d'un corn-steep pulvérulent sans support de séchage, contenant de l'acide lactique.

Le corn-steep pulvérulent conforme à l'invention est susceptible d'être obtenu en procédant à un enrichissement d'un corn-steep liquide en au moins un sel de métal, préalablement à une atomisation.

Il a été constaté, contre toute attente, qu'une telle étape d'enrichissement permettait l'atomisation du corn-steep liquide sans générer de problèmes de collage ou de mottage, et sans éliminer au préalable l'acide lactique qu'il contient.

En effet, la Demanderesse a démontré que les problèmes de séchage rencontrés dans les procédés de l'art antérieur étaient dus au fait que le lactate de potassium présent en grandes -quantités dans le corn-steep était incristallisable. En enrichissant le corn-steep en sel de métal, on déplace par effet de sel le lactate de potassium vers une forme cristallisable de lactate. Le corn-steep ainsi enrichi est donc facilement atomisable, sans qu'il soit nécessaire d'ajouter un support de séchage, ou d'éliminer l'acide lactique ou autre composé constitutif du corn-steep.

Le corn-steep pulvérulent conforme à l'invention est susceptible d'être obtenu selon une multitude de variantes mais tout particulièrement selon un procédé comportant les étapes suivantes :
- enrichissement du corn-steep liquide en au moins un sel de métal,
- atomisation du corn-steep liquide enrichi,
- récupération du corn-steep pulvérulent ainsi obtenu.

En ce qui concerne l'enrichissement du corn-steep liquide, on préfère que le sel de métal soit choisi dans le groupe constitué des sels de zinc, de magnésium, de calcium, de strontium, de baryum et de lithium. Selon un mode de réalisation préféré de l'invention, on choisit l'oxyde de magnésium. A titre indicatif, on ajoute au corn-steep liquide une quantité de MgO d'environ 3% en poids sec exprimé par rapport à la matière sèche du corn-steep liquide (sec/sec).

Le corn-steep liquide mis en oeuvre dans le procédé conforme à l'invention peut être notamment celui décrit dans le brevet US 4.359.528 ou encore ceux décrits dans les demandes de brevet EP 724.841 et EP 819.702 dont la Demanderesse est titulaire.

En ce qui concerne l'atomisation du corn-steep enrichi en sel de métal, on peut utiliser tout type de matériel connu de l'homme de métier.

La Demanderesse a démontré que l'on pouvait avantageusement fabriquer le corn-steep pulvérulent conforme à l'invention, par exemple en utilisant une tour d'atomisation de type NIRO.

On peut choisir avantageusement une température d'entrée d'air comprise entre 150 et 250°C, et des débits en matières entrantes tels que la température de l'air sortant de la tour soit comprise entre 80 et 150°C.

Le corn-steep pulvérulent obtenu selon le procédé conforme à l'invention peut être par la suite mis sous forme de comprimés par exemple sur presse alternative en présence de lubrifiant.

Le corn-steep pulvérulent conforme à l'invention peut avantageusement être employé en tant que substance nutritive dans la préparation de milieux de culture pour l'industrie de la fermentation. Il peut également être utilisé dans les domaines de l'alimentation, de la nutrition animale ou autres.

Contrairement aux corn-steep pulvérulents de l'art antérieur, le corn-steep conforme à l'invention conserve ses caractéristiques biochimiques initiales. De plus, il possède la caractéristique avantageuse d'être compressible, ce qui est particulièrement intéressant dans la pratique industrielle, en raison des risques et inconvénients liés à la manipulation des poudres. D'autre part, la forme comprimé permet un dosage minutieux dans la préparation de milieux de culture.

L'invention sera mieux encore comprise à l'aide des exemples qui suivent, lesquels ne se veulent pas limitatifs et font seulement état de certains modes de réalisations et de certaines propriétés avantageuses du corn-steep, pulvérulent selon l'invention.

### Exemple 1

### Préparation de corn-steep pulvérulent selon l'invention.

on prépare un corn-steep liquide à 50% de matière sèche en mettant en oeuvre le procédé décrit dans le brevet EP-A-0.819.702 au nom de la Demanderesse. On ajoute à celui-ci 3,2% en poids (sec/sec) de MgO sous forme d'une suspension à 100g/l dans une cuve thermostatée à 50°C, sous agitation.

Le pH du corn-steep liquide enrichi en MgO est de 5,7.
on procède ensuite à l'atomisation dans une tour NIRO dans les conditions suivantes :
- température d'entrée 200°C,
- température de sortie 96°C,
- vitesse de turbine : 15.000 tours/minute,
- capacité d évaporation : 80 litres/heure.

La teneur en acide lactique du corn-steep obtenu est de 14% en poids.

Les particules ne collent pas sur les parois de la chambre d'atomisation.

La poudre est dense et descend rapidement du cyclone.

### Exemple 2

### Application du corn-steep pulvérulent selon l'invention à la croissance des organismes.

L'étude décrite ci-après consiste à suivre l'augmentation du nombre des cellules en fonction du temps, dans un milieu de culture contenant une concentration donnée du corn-steep pulvérulent selon l'invention. Ceci est fait en comparaison à des milieux de culture contenant la même équivalence en azote apportée par d'autres sources.

L'étude concerne la croissance d'une souche de Saccharomyces cerevisiae. L'analyse des moûts de culture est effectuée à 0, 24 et 48 heures d'incubation par détermination de la mortalité cellulaire par cytométrie de flux (CHEMFLOW CHEMUNEX).

On prépare un milieu de préculture par addition dans de l'eau déminéralisée de 50g/l de glucose, 5g/l de corn-steep liquide à 50% de matière sèche, 5g/l de peptone, 3g/l de KHPO₄, et 1g/l de MgSO₄, 7H₂0. Le milieu est stérilisé 20 minutes à 120°C après ajustement du pH à 5,7. 50 ml de milieu sont ensemencés par une ose de cellules provenant d'un milieu nutritif gélosé CGA (MERCK).

La préculture est incubée 24 heures à 30°C sous agitation à 250 rotations par minute.

On prépare un milieu de culture par addition dans de l'eau déminéralisée de 100g/l de glucose et 5g/l de corn-steep pulvérulent conforme à l'invention.

Comparativement, on prépare des milieux de culture à 10g/l de corn-steep liquide à 50% de matière sèche, 10g/l de corn-steep liquide hydrolysé non atomisé (à 50% de MS), tel que décrit dans le brevet EP-A-819.702 au nom de la Demanderesse, ou 3g/l d'extrait de levure (YEAST EXTRACT commercialisé par la société DIFCO). Le volume du milieu est de 50ml dans des erlenmeyers de 500ml avec 4 chicanes dans le fond. Ces milieux sont stérilisés 20 minutes à 120°C.

On ensemence chacun de ces milieux avec 5 ml d'une même préculture de S. cerevisae. L'incubation est réalisée à 30°C sous agitation à 250 rpm pendant 48 heures.

L'analyse par cytométrie de flux se fait selon le protocole établi par la Société CHEMUNEX pour l'utilisation de l'appareil Chem Flow Auto System 3.

### Résultats :

Les résultats sont exprimés en pourcentage de cellules mortes présentes dans la population totale (estimée à 10⁸ cfu/g)

| | 0 H | 24 H | 48H |
|---|---|---|---|
| CSL (*) | 41,8% | 11,4% | 12,9% |
| CSL hydrolysé non atomisé | 41,8% | 10,5% | 11,4% |
| CS de l'invention | 41,8% | 11,2% | 11,8% |
| Extrait de levure | 41,8% | 46,2% | 56,4% |

| | | | |
|---|---|---|---|
| * Corn-steep liquide | | | |

Les résultats montrent un meilleur maintien de la viabilité cellulaire pour des cellules cultivées sur corn-steep atomisé pulvérulent selon l'invention, par rapport aux cellules cultivées sur extrait de levure.

Ces mêmes résultats montrent, pour des cellules cultivées sur le corn-steep conforme à l'invention, un maintien de la viabilité cellulaire similaire à celui obtenu pour des cellules cultivées sur du corn-steep liquide, hydrolysé ou non.

L'atomisation n'a donc altéré en rien les qualités nutritives du corn-steep. De plus, la densité importante de la poudre permet une manutention plus aisée que l'extrait de levure lors de la pesée notamment.

## Revendications

1. Corn-steep pulvérulent sans support de séchage caractérisé en ce qu'il contient de l'acide lactique.

2. Corn-steep pulvérulent selon la revendication 1 caractérisé en ce que sa teneur en acide lactique est supérieure ou égale à 5% en poids sur sec et de préférence supérieure ou égale à 10% en poids sur sec.

3. Corn-steep pulvérulent selon l'une ou l'autre des revendications 1 et 2 caractérisé en ce qu'il présente une teneur en ions métalliques comprise entre 1 et 10%, et de préférence comprise entre 2 et 5% en poids (sec/sec).

4. Corn-steep pulvérulent selon l'une quelconque des revendications 1 à 3 caractérisé en ce que les ions métalliques sont choisis dans le groupe constitué des sels de zinc, magnésium, calcium, strontium, baryum, lithium, seuls ou en mélange entre eux.

5. Corn-steep pulvérulent selon l'une quelconque des revendications 1 à 4 caractérisé en ce que les ions métalliques sont des sels de magnésium.

6. Procédé de préparation d'un corn-steep pulvérulent selon l'une quelconque des revendications 1 à 5 caractérisé en ce qu'il comprend les étapes suivantes :
a) enrichissement du corn-steep liquide en au moins un sel de métal,
b) atomisation du corn-steep liquide enrichi,
c) récupération du corn-steep pulvérulent ainsi obtenu.

7. Procédé selon la revendication 6 caractérisé en ce que le sel de métal est choisi dans le groupe constitué des sels de zinc, magnésium, calcium, strontium, baryum, lithium, seuls ou en mélange entre eux.

8. Procédé selon la revendication 7 caractérisé en ce que le sel de métal est l'oxyde de magnésium.

9. Comprimé caractérisé en ce qu'il comprend un corn-steep pulvérulent selon l'une quelconque des revendications 1 à 5.

10. Utilisation du corn-steep pulvérulent selon l'une quelconque des revendications 1 à 5, ou selon la revendication 9, en tant que substance nutritive dans l'industrie de la fermentation.
